# EUROPEAN PATENT APPLICATION

(11) **EP 4 620 441 A1**
(43) Date of publication of application: **24.09.2025**
(21) Application number: 23891206.7
(22) Date of filing: 02.10.2023
(51) Int. Cl.: A61F 13/15

(54) **INDIVIDUALLY-PACKAGED ABSORBENT ARTICLE AND PACKAGING SHEET**

(30) Priority: 18.11.2022 JP 2022185095
(71) Applicant: DAIO PAPER CORPORATION, Shikokuchuo-shi Ehime 799-0492 (JP)
(72) Inventor: YOSHIOKA, Chisaki, Sakura-shi, Tochigi 329-1411 (JP)
(74) Representative: Epping - Hermann - Fischer
(86) International application number: PCT/JP2023/035916
(87) International publication number: WO 2024/106048

(57) **Abstract**

An individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed and form a seal part, the packaging sheet has tensile strength in the longitudinal direction that is 15 N/25 mm or less, and when peeling strength in the seal part between different portions of the packaging sheet is referred to as Ss and the tensile strength of the packaging sheet is referred to as **Sw,** Ss < Sw is satisfied.

## Description

### TECHNICAL FIELD

The disclosures herein relate to individually packaged absorbent articles and packaging sheets.

### BACKGROUND ART

Generally, absorbent articles such as sanitary napkins, pantyliners, incontinence pads, and the like, are provided as individually packaged absorbent articles (individual packaging) in a state that they are individually packaged and sealed with packaging sheets for reasons such as hygiene in storage and convenience in carrying. Generally, as a packaging structure of the individually packaged absorbent article, for example, an absorbent article is disposed on the inner surface of the packaging sheet, the packaging sheet is then inwardly folded with the absorbent article, and both edge portions are sealed.

Resin films and nonwoven fabrics are still main materials of packaging sheets, but paper packaging sheets are also known (e.g., Patent Literature (PTL) 1).

### CITATION LIST

### PATENT LITERATURE

[PTL 1] Japanese Patent Laid-Open Publication No. 2022-24624

### SUMMARY OF THE INVENTION

### PROBLEMS TO BE SOLVED BY THE INVENTION

Packaging sheets made of paper have attracted attention in recent years from viewpoints such as Sustainable Development Goals. However, due to paper being more prone to tearing than other materials, practical applications of paper packaging sheets have not much advanced. Here, in order to prevent the paper packaging sheet from tearing, it is conceivable to use a strong packaging sheet having weight and/or a large thickness, for example, but such a strong packaging sheet tends to generate a rustling sound, for example, when carried or opened, and may not be disliked by users.

Therefore, there is a need for a configuration in which a sound does not tend to be generated when the packaging sheet is touched and the packaging sheet is resistant to tearing when a seal of the individually packaged absorbent article is peeled off.

In view of the above, an object of the present invention is to obtain the configuration in which a sound does not tend to be generated when the packaging sheet is touched and the packaging sheet is resistant to tearing upon opening, in the individually packaged absorbent article using the paper packaging sheet.

### MEANS OF SOLVING THE PROBLEM

One embodiment of the present invention is an individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed and form a seal part, the packaging sheet has tensile strength in the longitudinal direction that is 15 N/25 mm or less, and when peeling strength in the seal part between different portions of the packaging sheet is referred to as Ss and the tensile strength of the packaging sheet is referred to as Sw, Ss < Sw is satisfied.

### EFFECTS OF THE INVENTION

According to one embodiment of the present invention, in an individually packaged absorbent article using a paper packaging sheet, it is possible to obtain a structure in which sound does not tend to be generated when the packaging sheet is touched and the packaging sheet is resistant to tearing upon opening.

### BRIEF DESCRIPTION OF THE DRAWINGS

[FIG. 1] FIG. 1 is a plan view of an individually packaged absorbent article according to one embodiment of the present invention.
[FIG. 2] FIG. 2 is a plan view of the unfolded individually packaged absorbent article of FIG. 1.
[FIG. 3] FIG. 3 is a cross section of a line I-I of FIG. **1****.**
[FIG. 4] FIG. 4 is an enlarged view of a portion II of FIG. 1.
[FIG. 5] FIG. 5 is a drawing describing manufacturing of the individually packaged absorbent article.
[FIG. 6] FIG. 6 is a drawing describing a peeling strength test of a fastening tape in an example.

### DESCRIPTION OF THE PREFERRED EMBODIMENTS

In the following, embodiments of the present invention will be described with reference to the accompanying drawings. In the drawings, the same or corresponding constituent elements are denoted with the same reference numerals, and description about the elements may be omitted unless particularly described.

### <BASIC STRUCTURE OF INDIVIDUALLY PACKAGED ABSORBENT ARTICLE>

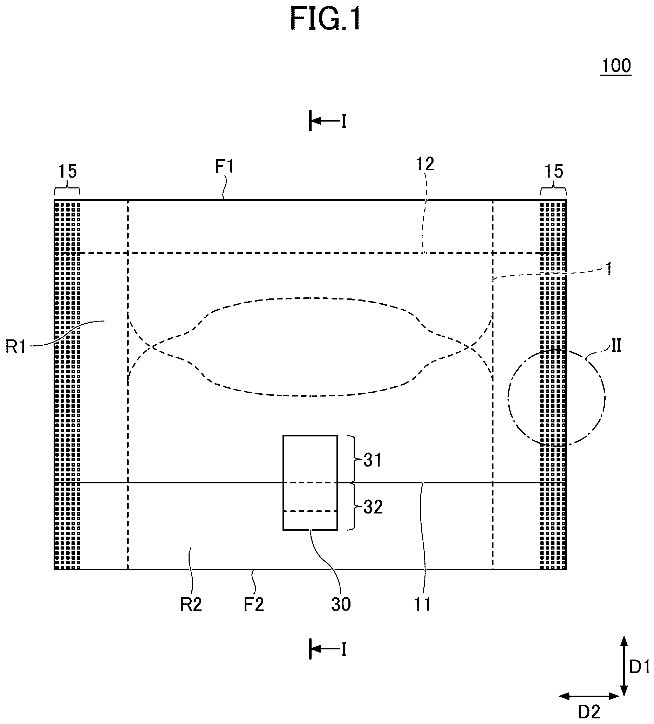

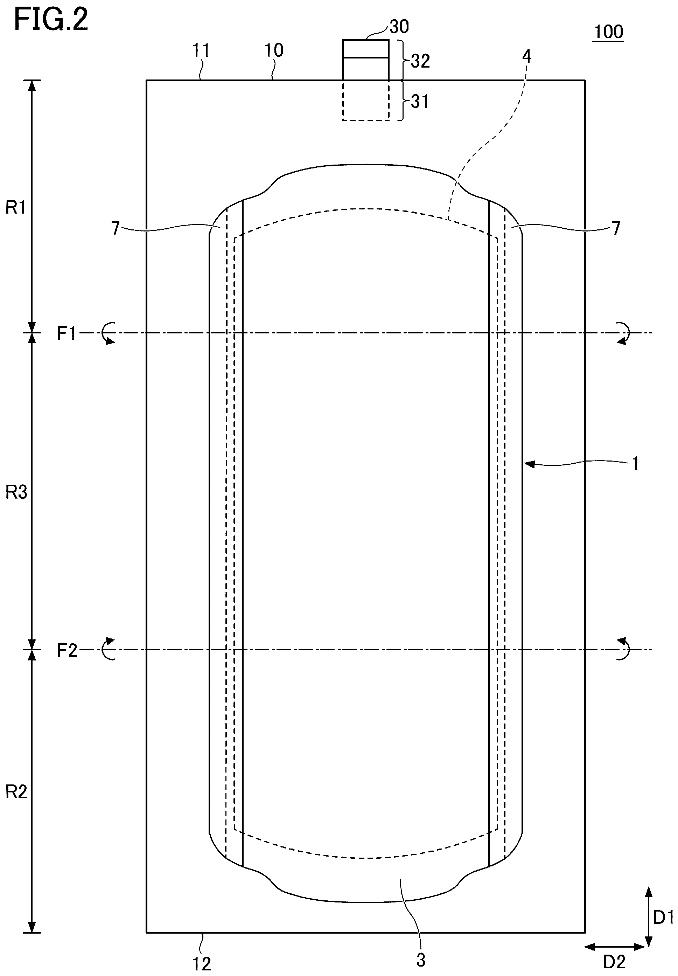

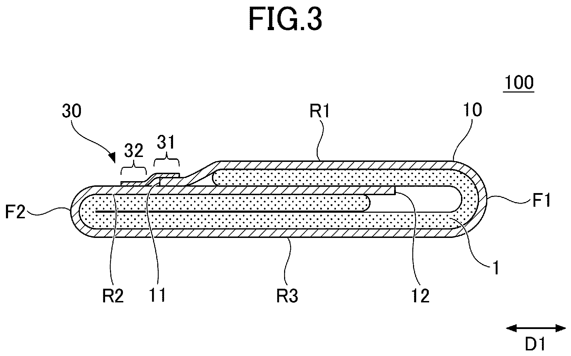

First, a basic structure of an individually packaged absorbent article will be described. FIG. 1 is a plan view of the individually packaged absorbent article 100. FIG. 2 is a plan view of the unfolded individually packaged absorbent article of FIG. 1, which is a drawing viewed from the inside of a packaging sheet 10 or from a surface of an absorbent article 1 facing a skin. FIG. 3 is a cross section of a line I-I of FIG. 1.

As shown in FIGS. 1 to 3, the individually packaged absorbent article 100 includes the packaging sheet 10 and the absorbent article 1 individually packaged by the packaging sheet 10. As shown in FIG. 2, the packaging sheet 10 may have an elongated shape when unfolded, and has a longitudinal direction (vertical direction) D1 and a transverse direction (horizontal direction) D2 perpendicular to the longitudinal direction. The longitudinal direction D1 and the transverse direction D2 of the packaging sheet 10 also correspond to the longitudinal direction and the transverse direction of the absorbent article 1, respectively. In a state of the individually packaged absorbent article 100, the longitudinal direction D1 of the packaging sheet 10 may be referred to as an opening direction and the transverse direction D2 may be referred to as a width direction.

### (ABSORBENT ARTICLE)

The absorbent article 1 packaged by the packaging sheet 10 may be a flat and elongated article to be attached so as to face an outlet of body fluid (menstrual blood, vaginal discharge, urine, etc.). Specific examples of the absorbent article 1 may be sanitary napkins, pantyliners, light incontinence pads, etc. The present description is mainly based on an example in which the absorbent article is a sanitary napkin.

As shown in FIG. 2, for example, the absorbent article 1 may have a liquid-permeable top sheet 3, a liquid-impermeable back sheet (not shown), and an absorber 4 disposed between the top sheet 3 and the back sheet.

For the back sheet, at least a water-resistant sheet material such as an olefin resin sheet, including polyethylene or polypropylene, can be used. A laminated nonwoven fabric in which a nonwoven fabric is laminated on a polyethylene sheet or the like, or a laminated sheet of a nonwoven fabric in which a waterproof film is interposed so as to substantially ensure a liquid impermeability, can be used. In addition, a material having moisture permeability may also be used.

As the top sheet 3, a porous or non-porous nonwoven fabric or a porous plastic sheet or the like is suitably used. As the material fibers constituting the nonwoven fabric, for example, olefins such as polyethylene and polypropylene, synthetic fibers such as polyester and polyamide, regenerated fibers such as rayon and cupra, and mixed fibers of the aforementioned, and natural fibers such as cotton can be used alone or in combination of two or more types.

The absorber 4 is not limited as long as it is a material capable of absorbing and retaining body fluids, but preferably includes a cotton-like pulp and a water-absorbent polymer. Superabsorbent polymer (SAP), superabsorbent fiber (SAF), and a combination of SAP and SAF can be used as the water-absorbent polymer. Examples of pulp include cellulose fibers such as chemical pulp and dissolving pulp obtained from wood, as well as artificial cellulose fibers such as rayon and acetate. The pulp may be hardwood pulp obtained from hardwood, softwood pulp obtained from softwood, or a mixture of the hardwood pulp and the softwood pulp. Moreover, the pulp may be recycled pulp recycled from used pulp.

Thickness of the absorber 4 may be 0.5 to 25 mm. The absorber 4 may be designed with an inflated structure in a region corresponding to a body fluid outlet (body fluid outlet corresponding region) and a region facing a buttock groove behind the body fluid outlet corresponding region. The absorber 4 has a size and shape that do not protrude from the top sheet 3 and back sheet. At the front and rear end edge portions of the absorber, the outer edges of the back sheet and top sheet 3 are bonded using adhesives such as hot melt, or bonding methods such as heat sealing or ultrasonic sealing.

As shown in FIG. 2, on the lateral outside of the absorber 4, side sheets 7, 7 may be provided along the longitudinal direction D1 at both ends of the transverse direction D2. A water-repellent nonwoven fabric or a hydrophilic nonwoven fabric can be used as the side sheet 7.

In the example shown in FIGS. 1 to 3, the absorbent article 1 has no wings, or is what is called a wingless type, but it may be constructed as a winged article having wings extending in the both lateral directions. The wings may be formed by joining the side sheet and the back sheet.

Further, an anti-slip adhesive part to prevent the absorbent article 1 from slipping from underwear when attached may be provided on a surface facing the back sheet (a non-skin surface, or the surface facing the underwear when attached) of the absorbent article 1. The anti-slip adhesive part may be formed in a plurality of strips extending in the longitudinal direction D1 or the transverse direction D2.

A total length of the absorbent article 1 may be 140 to 430 mm, and the width of the absorbent article 1 (when having wings, the width of the body excluding the wings) may be 40 to 130 mm.

### (PACKAGING SHEET)

The packaging sheet 10 in the present embodiment is made of paper. Using a paper packaging sheet 10 can contribute to reduction of plastics and achievement of Sustainable Development Goals. In addition, paper can give a unique texture, for example, an appearance and touch of a gentle impression of a natural material. In the present description, paper refers to vegetable fibers or other fibers bonded with a binding agent to form a thin, flat sheet. In particular, paper made of vegetable fibers as a main raw material, for example, can refer to those containing 50% or more of vegetable fibers, especially cellulose fibers, preferably 80% or more. The vegetable fibers (pulp) used as the raw material of paper may include wood pulp, non-wood pulp, and waste paper pulp, and may be either mechanical pulp or chemical pulp. The pulp may be pulp recycled from components of absorbent articles and/or packaging sheets for absorbent articles. Further, additives may be added to the paper. Examples of paper include various types of paper such as Western paper, Japanese paper, converted paper, and synthetic paper. Furthermore, paper conventionally used for other purposes, such as newsprint paper, printing paper (including wood free paper), writing paper, drawing paper, packaging paper, thin paper, hybrid paper, and the like may be used. In the case of thin paper, thin Japanese vellum, Indian paper, rice paper, glassine paper, tissue paper, toilet paper, filter paper, and the like may be used.

In the present description, a term "paper packaging sheet" mainly refers to a sheet containing the above-mentioned paper. The paper packaging sheet may include not only a packaging sheet made of only paper, but also a laminated sheet in which paper and a sheet made of a material other than paper are laminated. When the packaging sheet 10 includes a sheet made of a material other than paper, the material other than paper may be a resin film, nonwoven fabric, or the like. However, when the packaging sheet 10 is made of only paper, it is particularly preferable from the viewpoint of reducing plastic and/or giving a natural texture unique to paper to the product.

The paper packaging sheet 10 may be subjected to some processing. This processing may include, for example, crepe finishing, embossing, calendering, water-repellent finishing, slit processing, ply processing, printing processing, and the like. Strength and/or flexibility of the paper can be improved by crepe finishing or embossing. An antifouling function can be enhanced by water-repellent finishing (e.g., a water-repellent containing a silicone resin, a paraffin resin, a fluororesin, or the like is applied). In addition, when water-repellent finishing is performed on the back surface of the absorbent article 1, which is opposite to the anti-slip adhesive part, the anti-slip adhesive part can be brought into contact with the packaging sheet 10 without a release sheet.

The size of the packaging sheet 10 depends on the size and shape of the absorbent article 1 to be packaged. For example, when the packaging sheet 10 is fully spread out (not folded back), a length of the longitudinal direction D1 (sometimes referred to simply as length) can be set to 100 to 450 mm, and a length of the transverse direction D2 (sometimes referred to simply as width) can be set to 70 to 250 mm. In the illustrated example, the packaging sheet 10 has a rectangular shape when it is unfolded, but may have a shape such as an oblong shape, for example.

### <PACKAGING STRUCTURE OF INDIVIDUALLY PACKAGED ABSORBENT ARTICLE>

As shown in FIGS. 1 to 3, the absorbent article 1 is folded and packaged with the packaging sheet 10 to form an individually packaged absorbent article 100. At the time of folding, as shown in FIG. 2, the absorbent article 1 is arranged on the inner surface of the packaging sheet 10 so that the back sheet of the absorbent article 1 faces the inner surface of the packaging sheet 10. Both the packaging sheet 10 and the absorbent article 1 are folded in the longitudinal direction D1 at a first folding line F1 and a second folding line F2 along the width direction D2. More specifically, at the first folding line F1, a first region R1 including one end 11 of the longitudinal direction D1 of the packaging sheet 10 is folded back in the longitudinal direction D1, and at the second folding line F2, a second region R2 including the other end 12 of the longitudinal direction D1 of the packaging sheet 10 is folded back. The region between the first region R1 and the second region R2 of the packaging sheet 10 is a third region R3. In the illustrated example, the packaging sheet 10 is folded so that the second region R2 is folded first and the first region R1 overlaps the outer surface of the second region R2 (FIGS. 1 and 2), but the folding order of the first region R1 and the second region R2 may be reversed. After packaging the absorbent article 1 by inwardly tri-folding the packaging sheet 10 (folding the packaging sheet 10 in three) with the absorbent article 1, both edge portions of the transverse direction D2 are sealed along the longitudinal direction D1, and seal parts 15, 15 are formed.

Such a packaging form folded in three or more can be relatively easily formed, and the absorbent article 1 can be packaged hygienically, and the absorbent article 1 can be easily removed. The folding form is not limited to folding in three; it can also be folded in four or more, or in two.

### (SEAL PART)

As shown in FIG. 1, in the individually packaged absorbent article 100, both edges of the transverse direction D2 where the absorbent article 1 is not present are sealed, and seal parts 15, 15 are formed. The seal parts 15, 15 may be formed over the entire length of the longitudinal direction D1 of the individually packaged absorbent article 100. This is preferable from the viewpoint that it is possible to prevent dirt, dust, fingers or small objects from entering from the end edge in the transverse direction (sealing property is obtained). The seal parts 15, 15 may be formed by, for example, compression bonding different portions of the packaging sheet 10 in the thickness direction by inserting into a pair of rolls and applying pressure by both rolls (described later).

A range of the transverse direction D2 provided with the seal part 15 may be within a range of 20 mm from the end edge of the transverse direction D2. The seal part 15 may be formed in the entire range, or in a part of the range, for example, at a position away from the end edge of the transverse direction D2. The length (width) of the transverse direction D2 of the seal part 15 is preferably 3 to 15 mm.

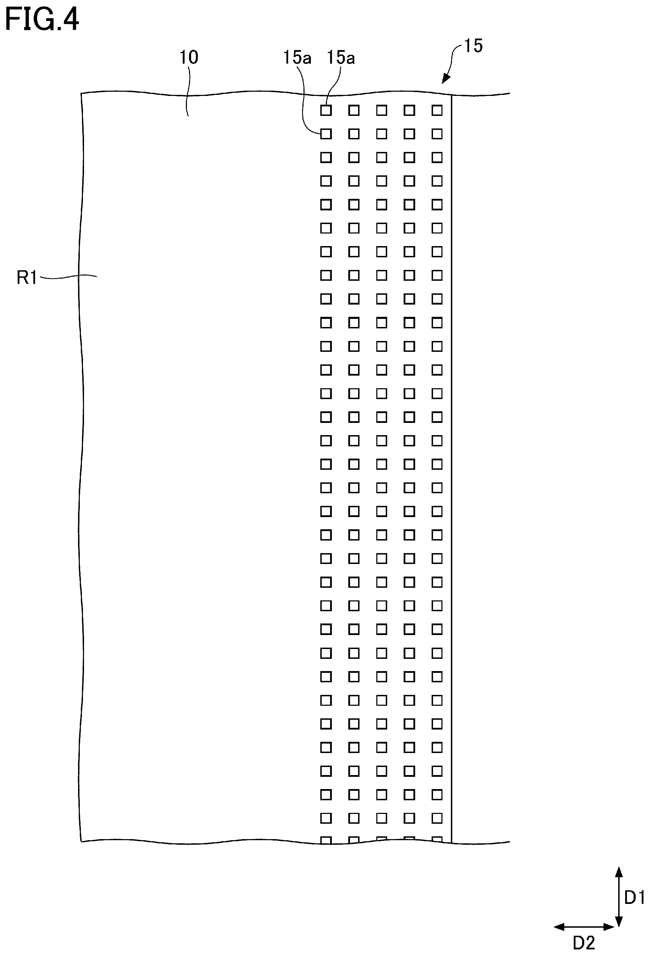

FIG. 4 is an enlarged view of a portion II including the seal part 15. As shown in FIG. 4, the seal part 15 may include a plurality of compression bonded parts (or joints) 15a, 15a, ... which are spaced apart from each other in planar view. The respective compression bonded joint parts 15a, 15a, ... are portions where the different portions of the packaging sheet 10 are compression bonded in the thickness direction. In the illustrated example, portions other than the plurality of compression bonded parts 15a, 15a, ... are non-joined parts where the different portions of the packaging sheet 10 are not joined to each other. Thus, because the compression bonded parts 15a, 15a, **...** are spaced apart from each other, it becomes easier to peel off the packaging sheets during opening, improving the opening ease. This is in contrast to when the different portions of the packaging sheet 10 are continuously bonded over the entire surface of the seal part 15. Additionally, this design prevents the edge portions of the individually packaged absorbent article 100 from becoming excessively hard and compromising the touch.

The plan view shape of each one of the compression bonded parts 15a, 15a, ... is a square in the example shown in FIG. 4, but it is not limited to the illustrated shape shown, and it may be, for example, a quadrilateral other than a square such as a rectangle, a parallelogram, a polygon other than a quadrilateral, a circle, an oval, a heart, a star, a drop, and the like. In the seal part 15, the size of one of the compression bonded parts 15a may be a square having sides of 0.2 to 2.5 mm or a size having an area equivalent to the aforementioned square. Moreover, arrangement of the plurality of compression bonded parts 15a, 15a, ... may be in a grid pattern as shown in FIG. 4, or a staggered pattern.

### (FASTENING TAPE)

As shown in FIG. 1, and the like, the individually packaged absorbent article 100 may be provided with a fastening tape 30 in the center of the transverse direction D2, near the end edge of the first region R1 (one end 11 of the packaging sheet 10). As shown in FIG. 1, the fastening tape 30 may be provided across both the first region R1 and the second region R2, straddling the one end 11 of the packaging sheet 10. In this specification, the portion of the fastening tape 30 adhered to the first region R1 in a plan view is referred to as a base 31, and the portion adhered to the second region R2 is referred to as a tip 32.

By providing the fastening tape 30, the user can easily lift and pull the first region R1 to open the individually packaged absorbent article 100 by holding the fastening tape 30 (more specifically, the tip 32 of the fastening tape 30), making the opening process more convenient. Moreover, by providing the fastening tape 30, when the absorbent article is replaced, after the used absorbent article is wrapped with the packaging sheet obtained from a newly opened individually packaged absorbent article, the end of the packaging sheet can be fastened to another part of the packaging sheet. Thus, the used absorbent article can be prevented from being exposed by opening the packaging sheet, and the used absorbent article can be disposed of hygienically.

The width (length in the transverse direction D2) of the fastening tape 30 is preferably 5 to 30 mm, more preferably 10 to 20 mm. The length of the entire fastening tape 30 is preferably 10 to 50 mm, more preferably 15 to 40 mm.

### <PACKAGING SHEET STRENGTH, SEAL STRENGTH, AND PEELING STRENGTH OF FASTENING TAPE AND THEIR RELATION>

The basic characteristics such as the material of the packaging sheet 10 used in the present embodiment are as described above, but in the present embodiment, more specifically, as the packaging sheet 10, a material having a lower strength than the conventional material is used. For example, the tensile strength of the packaging sheet 10 may be 15 N/25 mm or less. The tensile strength of the packaging sheet may be more preferably 13 N/25 mm or less, more preferably 10 N/25 mm or less, more preferably 8 N/25 mm, and more preferably 5 N/25 mm. The upper limit of the tensile strength may be the tensile strength of the packaging sheet 10 at least in the longitudinal direction D1. That is, the packaging sheet 10 may have the tensile strength in the above range only in the longitudinal direction D1, or in both the longitudinal direction D1 and the transverse direction D2. By using the packaging sheet 10 having strength in the above range, softness of the packaging sheet 10 can be ensured, the rustling sound generated when the packaging sheet is touched is reduced, rough feeling when the packaging sheet 10 is touched is also reduced, and the touch is improved.

Furthermore, the tensile strength of the packaging sheet 10 is preferably 1 N/25 mm or more, more preferably 3 N/25 mm or more, and more preferably 4 N/25 mm or more in order to maintain its intended function as a packaging sheet. The lower limit of the tensile strength may also be the lower limit of the tensile strength of the packaging sheet 10 at least in the longitudinal direction D1. That is, the packaging sheet 10 may have the above range of tensile strength only in the longitudinal direction D1, or may have the above range of tensile strength in both the longitudinal direction D1 and the transverse direction D2.

The transverse direction D2 of the packaging sheet 10 (width direction in the individually packaged absorbent article) may be in a flow direction (MD direction or vertical line direction) at the time of manufacturing the packaging sheet, and the longitudinal direction D1 (opening direction in an individually packaged absorbent article) of the packaging sheet 10 may be in the lateral direction (CD direction or horizontal line direction) orthogonal to the flow direction (MD direction) at the time of manufacturing the packaging sheet.

The tensile strength of the packaging sheet can be measured by testing a sample having a predetermined width using a tensile testing machine (e.g., a tensile testing machine made by Orientec Co., Ltd.) commonly used in the present technical field.

During the opening operation of the individually packaged absorbent article 100, the folded packaging sheet 10 is unfolded outward in the longitudinal direction D1 (e.g., the first region R1 and the second region R2 in FIG. 1 are unfolded in this order), and at this time, seals of the seal parts 15, 15 at both edge portions in the transverse direction D2 are peeled off. At this time, if the seal strength of the seal part 15 is excessively high, the packaging sheet 10 may be torn before the seal is peeled off. Conversely, in the present embodiment, by setting the strength of the packaging sheet and the seal strength to a predetermined relation, the packaging sheet 10 is resistant to tearing when the packaging sheet 10 is unfolded and the seal is peeled off during the opening operation, especially when the first region R1 is peeled from the second region R2. More specifically, Sw (N) is the tensile strength of the packaging sheet at least in the longitudinal direction D1, and Ss (N) is peeling strength between the different portions of the packaging sheets at the seal part, where Ss < Sw. The tensile strength Sw of the packaging sheet 10 can be measured per 25 mm width, considering the width of the user's fingers gripping it during the opening operation. The seal strength (N) is the peeling strength when the seal is peeled off at one of the two edges, and can be adjusted according to the width of the seal part 15, the seal formation method, and the seal pattern (size, arrangement, etc. of the compression bonded part). The seal strength can also be measured as the peeling strength between the two packaging sheets using the tensile testing machine described above. When the individually packaged absorbent article 100 is opened, a tensile force is applied to the packaging sheet not only in a certain direction but also in various directions, and a force other than the tensile force is also applied. The tensile strength of the packaging sheet 10 in the longitudinal direction D1 can be used as an indication of the strength that can avoid tearing of the packaging sheet during opening, or the strength that can avoid detachment of the fastening tape.

Furthermore, a difference (Sw - Ss) between the tensile strength and the seal strength of the packaging sheet may preferably be 3 N or more, more preferably 3.5 N or more. As a result, the strength of the packaging sheet is high, and effects of preventing the packaging sheet 10 from tearing during opening can be further improved.

The seal strength Ss of one of the seal parts 15 may preferably be 0.05 N or more, more preferably 0.1 N or more. By setting the seal strength Ss in the above range, unintended opening before starting use can be prevented.

When the individually packaged absorbent article 100 is provided with the fastening tape 30 (FIG. 1, etc.), it is preferable that Ss < St when St is the peeling strength of the fastening tape 30 with respect to the outer surface of the packaging sheet. The peeling strength St of the fastening tape can also be measured using the tensile testing machine described above, and the peeling strength can be adjusted depending on the width of the fastening tape 30 and a type of adhesive used. The relation between the seal strength Ss and the peeling strength St of the fastening tape ensures that the fastening tape 30 is not detached due to being weaker than the seal strength when opening the individually packaged absorbent article 100 holding the fastening tape 30, that is, when the packaging sheet 10 is unfolded outward in the longitudinal direction D1 (when the first region R1 is peeled from the second region R2).

Furthermore, the difference between the peeling strength St of the fastening tape and the seal strength, or St - Ss, may preferably be 1 N or more. In view of the seal parts 15 and 15 are being formed on both edge portions of the individually packaged absorbent article 100, it is preferable that the peeling strength St of the fastening tape is more than twice the seal strength Ss (St > 2 × Ss), since the detachment of the fastening tape 30 can be more reliably avoided when the packaging sheet 10 is unfolded.

When the tensile strength Sw of the packaging sheet is compared with the peeling strength St of the fastening tape, it is preferable that St < Sw. Thus, if a sudden and excessively large force is applied to the fastening tape 30 due to jolts or the like when unfolding in the longitudinal direction D1 by holding the tip 32 and lifting the first region R1 during opening, or at the time or after the first region R1 is fully unfolded, the fastening tape can detach before the packaging sheet 10 tears, preventing the packaging sheet from tearing.

Furthermore, the difference between the tensile strength Sw of the packaging sheet and the peeling strength St of the fastening tape, or Sw - St may preferably be 0.1 N or more, more preferably 0.125 N or more, and further preferably 1 N or more. As a result, the effect of avoiding the material of the packaging sheet 10 remaining on the fastening tape 30 can be further improved. Sw - St may preferably be 2 N or less, more preferably 1.5 N or less. As a result, it is possible to prevent the peeling strength of the fastening tape 30 from becoming excessively weak and the fastening tape 30 from being easily detached even by a small force.

The thickness of the paper packaging sheet 10 (in the case of crepe paper, the thickness after crepe finishing) may preferably be 40 to 200 µm, more preferably 70 to 150 µm, and further preferably 90 to 150 µm. A weight of the packaging sheet 10 may preferably be 12 to 50 g/m², more preferably 15 to 40 g/m², and more preferably 15 to 35 g/m². Such a relatively thin packaging sheet and/or a small weight of the packaging sheet may be flexible, and noise generated during opening and carrying can be reduced. Moreover, the touch is good. According to the present embodiment, even when such a paper packaging sheet having a thin thickness and/or a small weight is used, it is possible to provide an individually packaged absorbent article having a packaging sheet 10 that is resistant to tearing upon opening due to a predetermined relation between the tensile strength Sw of the packaging sheet and the seal strength Ss.

### <MANUFACTURING OF INDIVIDUALLY PACKAGED ABSORBENT ARTICLE>

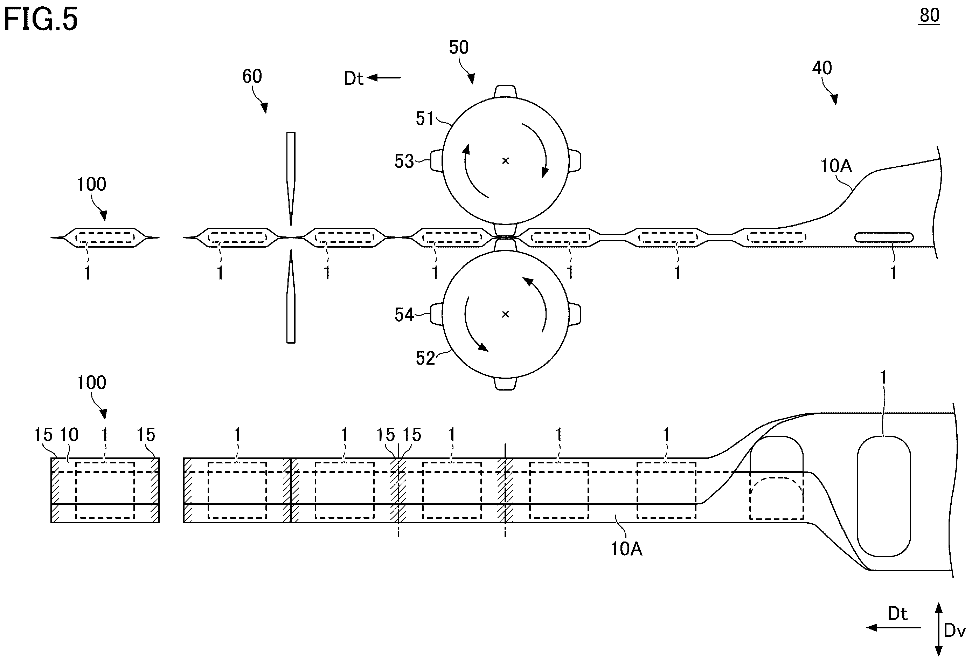

FIG. 5 shows a schematic diagram of an example of a manufacturing apparatus 80 of an individually packaged absorbent article 100. As shown in FIG. 5, a packaging sheet long body 10A to be a paper packaging sheet 10 (FIGS. 1 to 3) is transported in a conveyance direction Dt. The packaging sheet long body 10A can be obtained in a rolled-up state, and can be used by unrolling it from the roll. The packaging sheet long body 10A is transported along its longitudinal direction, and the absorbent articles 1, 1, ... are disposed on the inner surface of the packaging sheet long body 10A at intervals. The absorbent article 1 is disposed so that the longitudinal direction of the absorbent article 1 is along an orthogonal direction Dv orthogonal to the conveyance direction Dt. Subsequently, the packaging part 40 of the manufacturing apparatus 80 folds the packaging sheet long body 10A with the absorbent article 1 in the orthogonal direction Dv (i.e., the longitudinal direction D1 of the absorbent article 1) at a folding position along the conveyance direction Dt. It is preferable that the absorbent article 1 is inwardly folded in three or more so that the absorbent article 1 is not exposed after folding.

The absorbent articles 1, 1, ... disposed at intervals in the conveyance direction Dt are further conveyed to the seal forming part 50 of the apparatus 80 in a state of being packaged by the packaging sheet long body 10A. As shown in FIG. 5, the seal forming part 50 includes a pair of rolls 51, 52. In the seal forming part 50, at a position between the absorbent articles 1, 1, ... (a position where the absorbent article 1 is not disposed), different parts of the packaging sheet long body 10A stacked in the thickness direction are pressed in the thickness direction by the pair of rolls 51, 52 to form a seal part 15. In the seal forming part 50, seal parts 15, 15 (e.g., the seal part on the right of the individually packaged absorbent article downstream in the conveyance direction and the seal part on the left of the individually packaged absorbent article upstream in the conveyance direction) of two adjacent individually packaged absorbent articles are simultaneously formed.

Of the pair of rolls in the seal forming part 50, the first roll 51 may be provided with pressurizing parts 53, 53, ... along the circumferential direction, and similarly, the second roll 52 may be provided with pressurizing parts 54, 54, ... along the circumferential direction. Here, for example, the surfaces of the pressurizing parts 53, 53, ... of the first roll 51 may be provided with a plurality of protrusions that later correspond to the compression bonded parts 15a, 15a, ... (FIG. 4, etc.). Conversely, the surfaces of the pressurizing parts 54, 54, ... of the second roll 52 may be flat. By rotating the pair of rolls, the pressurizing part 53 of the first roll 51 and the pressurizing part 54 of the second roll 52 face each other, and the packaging sheet long body 10A sandwiched between the rolls is pressurized in the thickness direction. At this time, the temperature of the roll may be raised so that the packaging sheet long body 10A is heated.

After the seal part 15 is formed in the seal forming part 50, the packaging sheet long body 10A is cut at the center of the conveyance direction Dt in the region where the seal part is formed by a cutting part 60, so that the individually packaged absorbent article 100 having the seal parts 15, 15 at the edge portion of the conveyance direction Dt can be obtained. The seal forming part 50 and the cutting part 60 may be integrated means, that is, forming and cutting of the seal part may be performed by a single apparatus.

The conveyance direction Dt may be in a flow direction (MD direction or vertical line direction) during manufacturing the packaging sheet long body 10A, and the orthogonal direction Dv may be in the transverse direction (CD direction or horizontal line direction) orthogonal to the flow direction (MD direction). That is, the transverse direction D2 of the packaging sheet 10 in the individually packaged absorbent article 100 may be in the MD direction, and the longitudinal direction D1 of the packaging sheet 10 may be in the CD direction. However, the MD direction and the CD direction of the material in the individually packaged absorbent article 100 of the packaging sheet 10 are not limited to the above, and the transverse direction D2 of the packaging sheet 10 in the individually packaged absorbent article 100 may be in the CD direction, and the longitudinal direction D1 of the packaging sheet 10 may be in the MD direction.

### EXAMPLES

### [1] TEAR TEST OF PACKAGING SHEET DURING UNFOLDING (EXAMPLE 1-1 TO EXAMPLE 1-5)

Individually packaged absorbent articles in which a sanitary napkin (20.5 cm, with wings) was packaged by a packaging sheet were prepared. The packaging sheet was crepe paper (thickness: 90 µm, weight: 18 g/m²) produced mainly from hardwood pulp and softwood pulp, and had a size of 240 mm in length D1 in the longitudinal direction (opening direction) and 120 mm in length D2 in the transverse direction (width direction). More specifically, the individually packaged absorbent articles were arranged in such a way that the longitudinal direction of the sanitary napkin was along the longitudinal direction of the packaging sheet, and the packaging sheet and sanitary napkin were inwardly folded in three as shown in FIGS. 1 to 3, and both edges of the packaging sheet in the transverse direction were sealed. The seal part formed by the seal was a 6 mm wide seal part consisting of a plurality of compression bonding portions as shown in FIGS. 1 and 4 by compression bonding by a pair of rolls as shown in the seal forming part 50 in FIG. 5. A plurality of dot-shaped protrusions corresponding to the compression bonding portions were formed on one surface of the pair of rolls, and the remainder of the surface was flat. The compression bonding was performed under conditions of a roll temperature (set value) of 152°C and a line speed of 1050 sheets per minute in all cases. However, the seal strength of each obtained example was made different. In addition, a fastening tape (reference numeral 30 in FIGS. 1 and 3) was adhered over the longitudinal direction of the exposed end of the packaging sheet after folding. The fastening tape was 12 mm in width, and the peeling strength of the fastening tape with respect to the packaging sheet was 3.47 N/12 mm, which was sufficiently large for the seal strength of the seal part.

The individually packaged absorbent article of each example was evaluated from the viewpoint of tearing of the packaging sheet during unfolding. The tensile strength (N) of the packaging sheet used in the longitudinal direction was measured. The seal strength (N) of the seal part of each example was also measured. The seal strength (N) of the seal part of the individually packaged absorbent article was measured by preparing another individually packaged absorbent article manufactured in the same manner as the individually packaged absorbent article of the example to be measured, and evaluating the seal part of the other individually packaged absorbent article.

The methods of evaluation and measurement were as follows. Results are shown in Table 1.

### <PACKAGING SHEET TEAR EVALUATION (TEAR EVALUATION OF PACKAGING SHEET DURING UNFOLDING FIRST REGION)>

The individually packaged absorbent article in each example was unfolded in the longitudinal direction by a general method, specifically, holding the fastening tape (first region R1 in FIGS. 1 and 3 was unfolded). At this time, it was observed whether or not the packaging sheet tore when the seals at both edges in the transverse direction were peeled off. When the packaging sheet did not tear, it was designated as "o", when the packaging sheet tore slightly (5 mm or less), it was designated as "Δ", and when the packaging sheet tore significantly (more than 5 mm), it was designated as "×".

In the opening ease evaluation of Examples 1-1 to 1-5, the packaging sheet did not tear at the beginning of peeling of the fastening tape (when the fastening tape was peeled from the second region R2 (FIG. 1)), and the fastening tape was not detached.

### <TENSILE STRENGTH OF PACKAGING SHEET Sw>

Tensile strength of the packaging sheet in the longitudinal direction (opening direction in an individually packaged absorbent article) was measured. The packaging sheet was cut into a rectangular test piece of 120 mm in the longitudinal direction of the packaging sheet (opening direction in the individually packaged absorbent article) × 25 mm in the transverse direction of the packaging sheet (width direction in the individually packaged absorbent article), and the tensile test was conducted using a tensile testing machine (Tensilon testing machine "RTC-1210A", produced by Orientec Co., Ltd.) at a chuck interval of 50 mm and a tensile speed of 500 mm/min, and the tensile strength and the tensile elongation were measured. The tensile strength (N) of the packaging sheet was an average value of six measurements.

### <SEAL STRENGTH Ss>

The edge portion of the individually packaged absorbent article in the transverse direction was cut at a position of 25 mm from the end edge in the transverse direction, and a test was conducted using the above-mentioned tensile testing machine. Specifically, one chuck was attached after slightly peeling off the exposed longitudinal end edge of the packaging sheet (one longitudinal end 11 included in the first region R1 in FIG. 1), and the other chuck was attached near the longitudinal end edge of the individually packaged absorbent article (folded line F2 in FIG. 1). The chucks were pulled in opposite directions with a chuck interval of 10 mm and a tensile speed of 100 mm/min. The maximum point load (N) was taken as the seal strength (peeling strength) .

**[Table 1]**

| | Example 1-1 | Example 1-2 | Example 1-3 | Example 1-4 | Example 1-5 |
|---|---|---|---|---|---|
| Seal Strength Ss [N] | 0.14 | 0.24 | 0.37 | 1.51 | 1.82 |
| Tensile Strength of Packaging Sheet Sw [N] | 4.41 | 4.41 | 4.41 | 4.41 | 4.41 |
| Sw - Ss | 4.27 | 4.17 | 4.04 | 2.90 | 2.59 |
| St | 3.47 | 3.47 | 3.47 | 3.47 | 3.47 |
| St - Ss | 3.33 | 3.23 | 3.10 | 1.96 | 1.65 |
| Packaging Sheet Tear Evaluation (Tear Evaluation of Packaging Sheet during Unfolding First Region) | ○ | ○ | ○ | Δ | Δ |

As shown in Table 1, Example 1-1 to Example 1-5 satisfied Ss < Sw by comparing the seal strength Ss with the tensile strength Sw of the packaging sheet. Therefore, it was found that the individually packaged absorbent article satisfying this relation did not easily tear the packing sheet. In addition, when the difference between the seal strength Ss and the tensile strength Sw of the packaging sheet is 3 N or more (Example 1-1 to Example 1-3), it was found that the tear particularly does not readily occur.

### [2] PEELING TESTS OF FASTENING TAPES (EXAMPLE 2-1 TO EXAMPLE 2-7)

In the same manner as Example 1-1 to Example 1-5, individually packaged absorbent articles according to Example 2-1 to Example 2-7 were prepared. However, in all of Example 2-1 to Example 2-7, the seal strength was set to be equivalent (0.25 N) for convenience. In each example, the type of fastening tape was changed so that adhesive strength of the fastening tape was different.

The peeling strength (adhesive strength) (N) of the fastening tape used in each example was measured. In addition, material adhesion to the packaging sheet on the fastening tape side was also evaluated at the measurement. The present inventors considered that the evaluation of material adhesion to the fastening tape of the packaging sheet serves as one indicator of ease and difficulty of tearing the packaging sheet during opening. The evaluation and measurement were carried out as follows. The results are shown in Table 2.

### <PEELING STRENGTH OF FASTENING TAPE>

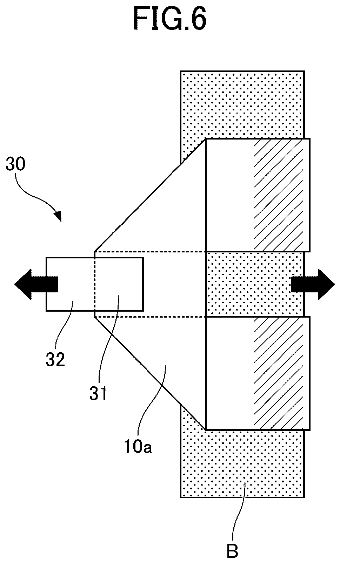

The portion to which the fastening tape was attached was cut out from the individually packaged absorbent article. Specifically, the tip of the fastening tape (portion of reference numeral 32 in FIG. 1, etc.) was peeled from the packaging sheet, the base of the fastening tape (portion of reference numeral 31 in FIG. **1****,** etc.) remained attached, and the packaging sheet was cut to 100 mm in the transverse direction (D2) and 20 mm in the longitudinal direction (D1), and the packaging sheet piece 10a was used as a test piece. Then, as shown in FIG. **6****,** the end part of the transverse direction D2 was folded back and attached to a mount B so that a ring was formed between the packaging sheet piece 10a and the mount B. The attaching region between the packaging sheet piece 10a and the mount B is indicated by a diagonal line in FIG. **6****.** Then, the mount B was held by one chuck and the tip of the fastening tape 30 was held by the other chuck and pulled in the opposite direction. This peeling strength test was conducted using a tensile testing machine (Tensilon testing machine "RTC1210", produced by Orientec **Co.,** Ltd.) at a chuck interval of 20 mm and a tensile speed of 300 mm/min.

### <MATERIAL ADHESION EVALUATION OF FASTENING TAPE ON PACKAGING SHEET>

In the above peeling test, the remaining material on the packaging sheet on the fastening tape was observed when the fastening tape was peeled off. When the remaining material on the packaging sheet was less than 0 to 30% of the area of the fastening tape, it was designated as "o", when it was 30% or more and less than 80%, it was designated as "Δ", and when it was more than 80%, it was designated as "×". Even in the case of "Δ", and even if the material adhered to the fastening tape, quality for opening as an individually packaged absorbent article is sufficient, and the fastening tape can be reused when the used absorbent article is wrapped with the packaging sheet when the absorbent article is replaced.

**[Table 2]**

| | Example 2-1 | Example 2-2 | Example 2-3 | Example 2-4 | Example 2-5 | Example 2-6 | Example 2-7 |
|---|---|---|---|---|---|---|---|
| Peeling Strength of Fastening Tape St [N] | 3.62 | 3.13 | 3.47 | 3.83 | 4.33 | 5.07 | 5.36 |
| Tensile Strength of Packaging Sheet Sw [N] | 4.41 | 4.41 | 4.41 | 4.41 | 4.41 | 4.41 | 4.41 |
| Sw - St | 0.79 | 1.28 | 0.94 | 0.58 | 0.08 | -0.66 | -0.95 |
| Material Adhesion Evaluation of Fastening Tape on Packaging Sheet | ○ | ○ | ○ | ○ | Δ | × | × |

In Table 2, when the peeling strength St (N) of a 12 mm width fastening tape was compared with the tensile strength (N) of a 25 mm width packaging sheet, it was found that material adhered to the fastening tape remarkably in the cases of St < Sw (Example 2-6 and Example 2-7). In addition, it was found that in examples satisfying St < Sw, material adhesion of the packaging sheet on the fastening tape is less likely to occur particularly when Sw - St is 0.1 N or more (Example 2-1 to Example 2-4).

### [3] DETACHMENT TEST OF FASTENING TAPE (EXAMPLE 3-1 AND EXAMPLE 3-2)

The present test was conducted to observe detachment of the fastening tape by changing the peeling strength St and seal strength Ss of the fastening tape. In the same manner as in Example 1-1 to Example 1-5 and Example 2-1 to Example 2-7, individually packaged absorbent articles according to Example 3-1 and Example 3-2 were prepared. However, in Example 3-1 and Example 3-2, the seal strength was made to be the same, while the type of fastening tape in each example was changed so that the adhesive strength of the fastening tape was different.

The individually packaged absorbent articles of each example were evaluated from the viewpoint of tendency toward detachment of the fastening tape. The results are shown in Table 3.

### <DETACHMENT EVALUATION OF FASTENING TAPE>

The individually packaged absorbent article in each example was unfolded in the longitudinal direction by the usual method, specifically, holding the fastening tape (the first region R1 in FIGS. 1 and 3 was unfolded to the end). At this time, the case where the fastening tape was not detached and unstuck at all was designated as "∘", the case where the fastening tape was not detached but partially unstuck was designated as "Δ", and the case where the fastening tape was detached was designated as "×".

**[Table 3]**

| | Example 3-1 | Example 3-2 |
|---|---|---|
| Seal Strength Ss [N] | 1.47 | 1.46 |
| Peeling Strength of Fastening Tape St [N] | 2.28 | 3.47 |
| Tensile Strength of Packaging Sheet Sw [N] | 4.41 | 4.41 |
| Sw - Ss | 2.28 | 3.47 |
| Sw - St | 2.13 | 0.94 |
| St - Ss | 0.81 | 2.01 |
| Detachment Evaluation of Fastening Tape | Δ | ○ |

As shown in Table 3, both of Example 3-1 and Example 3-2 satisfy Ss < St, but unsticking of the fastening tape was less likely to occur when the packaging sheet was unfolded when St - Ss was 1 N or more (Example 3-2).

Although the present invention has been described based on the embodiments described above, the present invention is not limited to these embodiments, and various variations and modifications may be made without departing from the scope of the present invention. Moreover, the above-described components can be combined optionally.

Further, specific aspects of the present invention will be described below.

### (Clause 1)

An aspect of Clause 1 is an individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed and form a seal part, the packaging sheet has tensile strength in the longitudinal direction that is 15 N/25 mm or less, and when peeling strength in the seal part between different portions of the packaging sheet is referred to as Ss and the tensile strength of the packaging sheet is referred to as **Sw,** Ss < Sw is satisfied.

According to the aspect according to Clause **1,** the packaging sheet has a relatively small predetermined tensile strength. Therefore, the softness of the packaging sheet is improved, the rustling sound generated when the packaging sheet is touched can be reduced, rough feeling when the packaging sheet is touched can also be reduced, and the touch is improved. Moreover, by the present embodiment, even in the packaging absorbent article using such a packaging sheet having a relatively small tensile strength, by setting the strength of the packaging sheet and the seal strength to a predetermined relation, the packaging sheet is resistant to tearing when unfolded and the seal is peeled off during opening.

### (Clause 2)

In an aspect of Clause 2, an exposed end of the packaging sheet in the longitudinal direction is fastened by a fastening tape, and when peeling strength between the fastening tape and the packaging sheet is referred to as St, Ss < St is satisfied.

According to the aspect according to Clause 2, the fastening tape facilitates holding the end of the packaging sheet at the time of opening, thereby facilitating opening. In addition, the fastening tape has an advantage that it can be used to fasten the packaging sheet after wrapping the used absorbent article when the used absorbent article is to be disposed of. Moreover, even in the configuration provided with the fastening tape in this manner, the predetermined relation between the seal strength of the seal part and the peeling strength of the fastening tape can ensure that the fastening tape is not detached when the seal of the seal part is peeled off.

### (Clause 3)

In an aspect of Clause 3, St < Sw is satisfied.

According to the aspect according to Clause **3,** even if a sudden and excessively large force is applied to the fastening tape due to jolts or the like when the packaging sheet is unfolded in the longitudinal direction D1 by holding the tip (one end in the longitudinal direction) of the packaging sheet (a region disposed outside) during opening, or at the time or after the packaging sheet is fully unfolded, the fastening tape can detach before the packaging sheet tears, preventing the packaging sheet from tearing.

### (Clause 4)

In an aspect of Clause 4, thickness of the packaging sheet is 40 to 200 µm.

According to the aspect according to Clause **4,** by using the relatively thin packaging sheet, the flexibility of the packaging sheet can be ensured, and the rustling sound when the packaging sheet is touched can be reduced and the touch can be improved. Moreover, by the present embodiment, even in the packaging absorbent article using such a relatively thin packaging sheet, by setting the strength of the packaging sheet and the seal strength to a predetermined relation, the packaging sheet is resistant to tearing when unfolded and the seal is peeled off during opening.

### (Clause 5)

An aspect of Clause 5 is a packaging sheet made of paper for individually packaging an absorbent article, wherein the packaging sheet is configured to form an individually packaged absorbent article by being inwardly folded in a longitudinal direction with the absorbent article disposed inside the packaging sheet, and having both edge portions in a transverse direction orthogonal to the longitudinal direction being sealed and forming a seal part, and when peeling strength in the seal part between different portions of the packaging sheet is referred to as Ss and tensile strength of the packaging sheet is referred to as Sw, Ss < Sw is satisfied.

According to the aspect of Clause 5, a packaging sheet having the same effect as that of Clause 1 can be obtained.

The present application is based on and claims priority to Japanese patent application no. 2022-185095 filed on November 18, 2022, with the Japanese Patent Office, the entire contents of which are hereby incorporated by reference.

### REFERENCE SIGNS LIST

1 absorbent article
3 top sheet
4 absorber
7 side sheet
10 packaging sheet
11 one end in the longitudinal direction
12 the other end in the longitudinal direction
15 seal part
15a compression bonded part
30 fastening tape
31 base
32 end
40 packaging part
50 seal forming part
60 cutting part
80 individually packaged absorbent article manufacturing apparatus
100 individually packaged absorbent article
D1 longitudinal direction of packaging sheet (opening direction)
D2 transverse direction of packaging sheet (width direction)
F1 first folding line
F2 second folding line
R1 first region
R2 second region
R3 third region

## Claims

1. An individually packaged absorbent article, for which an absorbent article is individually packaged by a packaging sheet made of paper, wherein:
the packaging sheet is inwardly folded in a longitudinal direction with the absorbent article disposed inside the packaging sheet, and both edge portions in a transverse direction orthogonal to the longitudinal direction are sealed and form a seal part;
the packaging sheet has tensile strength in the longitudinal direction that is 15 N/25 mm or less; and
when peeling strength in the seal part between different portions of the packaging sheet is referred to as Ss and the tensile strength of the packaging sheet is referred to as Sw, Ss < Sw is satisfied.

2. The individually packaged absorbent article according to claim 1, wherein:
an exposed end of the packaging sheet in the longitudinal direction is fastened by a fastening tape; and
when peeling strength between the fastening tape and the packaging sheet is referred to as St, Ss < St is satisfied.

3. The individually packaged absorbent article according to claim 2, wherein St < Sw is satisfied.

4. The individually packaged absorbent article according to claim 1 or **2,** wherein thickness of the packaging sheet is 40 to 200 µm.

5. A packaging sheet made of paper for individually packaging an absorbent article, wherein:
the packaging sheet is configured to form an individually packaged absorbent article by being inwardly folded in a longitudinal direction with the absorbent article disposed inside the packaging sheet, and having both edge portions in a transverse direction orthogonal to the longitudinal direction being sealed and forming a seal part; and
when peeling strength in the seal part between different portions of the packaging sheet is referred to as Ss and tensile strength of the packaging sheet is referred to as **Sw,** Ss < Sw is satisfied.
